# EUROPÄISCHE PATENTSCHRIFT

(11) **EP 2 836 266 B1**
(45) Veröffentlichungstag und Bekanntmachung des Hinweises auf die Patenterteilung: **14.09.2016**
(21) Anmeldenummer: 13720761.9
(22) Anmeldetag: 09.04.2013
(51) Int. Cl.: A61M 16/10, A61F 5/443, A61M 16/04, A61F 13/00, A61F 13/04

(54) **HALTERUNG FÜR SPRECHVENTILE UND/ODER WÄRME-FEUCHTIGKEITSTAUSCHER UND DAFÜR GEEIGNETE WÄRME-FEUCHTIGKEITSTAUSCHER**
MOUNTING FOR SPEECH VALVES AND/OR HEAT-MOISTURE EXCHANGERS AND HEAT-MOISTURE EXCHANGERS SUITABLE THEREFOR
SUPPORT POUR VALVES DE PHONATION ET/OU ÉCHANGEURS DE CHALEUR ET D'HUMIDITÉ ET ÉCHANGEURS DE CHALEUR ET D'HUMIDITÉ ADAPTÉS À CELUI-CI

(30) Priorität: 12.04.2012 DE 202012003723 U; 08.05.2012 DE 202012004539 U; 14.06.2012 DE 202012005851 U; 14.09.2012 DE 202012008906 U
(43) Veröffentlichungstag der Anmeldung: 18.02.2015
(73) Patentinhaber: Neubauer, Norbert, 38820 Halberstadt (DE)
(72) Erfinder: Neubauer, Norbert, 38820 Halberstadt (DE)
(74) Vertreter: Pfeiffer, Rolf-Gerd
(86) Internationale Anmeldenummer: PCT/DE2013/000189
(87) Internationale Veröffentlichungsnummer: WO 2013/152755

(56) Entgegenhaltungen:
- EP-A2- 0 387 220
- EP-A2- 1 787 671
- EP-B1- 0 959 927
- WO-A1-99/29268
- DE-U1-202008 002 602
- DE-U1-202011 003 781
- US-A- 4 325 366
- US-A1- 2003 029 456

## Beschreibung

Die Erfindung betrifft eine Halterung für Sprechventile und/oder Wärme-Feuchtigkeitstauscher und dafür geeignete Wärme-Feuchtigkeitstauscher, die bevorzugt bei Tracheostomapflastern Anwendung findet.
Nach einer Kehlkopfentfernung können verstärkt Atemwegsprobleme auftreten, weil aufgrund der unterbrochenen Verbindung zwischen Nase und Lunge die Funktionen der Nase entfallen. Die Atemluft wird nicht mehr gefiltert, angewärmt und befeuchtet. Dies führt zu Irritationen der Bronchialschleimhaut, übermäßiger Sekretproduktion und dadurch zu vermehrtem Husten. Die natürliche Nasenatmung bietet neben der Erwärmung, Befeuchtung und Filterung der Atemluft außerdem einen Atemwiderstand, der für die Sauerstoffanreicherung im Blut wichtig ist. Der geringere Widerstand nach der Kehlkopfentfernung lässt die Sauerstoffkonzentration im Blut sinken. Für den Betroffenen zeigt sich das in Form von Müdigkeit, Niedergeschlagenheit und verminderter Leistungsfähigkeit. Die Feucht-Wärme-Austauscher sind für Patienten mit spontaner Atmung vorgesehen und werden üblicherweise auf einen Universalkonus einer Trachealkanüle oder eines Tubus aufgesteckt und dienen zur pulmonalen Rehabilitation. Die Feucht-Wärme-Austauscher entziehen der ausgeatmeten Luft des Patienten die Feuchtigkeit und Wärme und führen diese über die eingeatmete Luft dem Patienten wieder zu.
Aus US 4,325,366 ist ein Sprechventil bekannt, das vermittels eines doppelseitig klebenden Pflasters um das Stoma eines Patienten befestigt wird, wobei das gesamte Ventil in eine trommelförmige Aufnahme eingesteckt wird. Dabei ist das Ventil einzig durch Klemmreibung in einer trommelförmigen Aufnahme lösbar fixiert.

Eine vergleichbare Aufnahme wird von der Fa. Heimomed Heinze GmbH & Co. KG unter der Bezeichnung PRIM-AIR-STRIP vertrieben, wobei dort ebenfalls austauschbare Sprechventile und/oder Wärme-Feuchtigkeitstauscher in eine ebenfalls trommelförmige elastische Aufnahme nahezu vollständig eingedrückt werden. Eine zusätzliche Nut, die in ein entsprechendes Gegenstück des das Ventil oder den Wärme-Feuchtigkeitstauscher tragenden Gehäuses eingreift, sorgt hier für eine zusätzliche Sicherung genannter Baugruppen gegen unbeabsichtigtes Lösen und dadurch für einen festeren Sitz.

Eine vergleichbare, aber praktisch doppelt soweit vom Stoma abragende Vorrichtung für einen ebenfalls vermittels eines doppelseitigen Klebebandes angebrachten Wärme-Feuchtigkeitstauscher ist in EP 0387220 B2 beschrieben. Dort wird ein Wärme-Feuchtigkeitstauscher auf den oberen Rand einer trichterförmig ausgebildeten und auf das Stoma aufgeklebten Haltevorrichtung mittels Krallen eingeschnappt. Diese Vorrichtung weist den Nachteil einer relativ hohen, vom Stoma beabstandeten Bauform auf und genügt damit nicht ästhetischen Gesichtspunkten des Trägers.

In WO 99/29268 ist eine Sprechventilanordnung beschrieben, bei der ein Gehäuse zur Aufnahme eines Wärme-Feuchtigkeitstauscher und eines Ventils vorgesehen ist. Dieses Gehäuse wird ebenfalls mit einer Haltevorrichtung, die in bekannter Weise auf das Stoma aufgeklebt ist verbunden, wobei hier, im Gegensatz zu den vorstehend beschriebenen Vorrichtungen, das gesamte, das Sprechventil und einen Wärme-Feuchtetauscher aufnehmende Gehäuse nur am unteren Rand dieses Gehäuses über eine zirkular das proximale Gehäuseende umlaufenden Rippe, die in eine nutförmige, am Klebeband befestigte Halterung eingreift, gehaltert ist. Bei dieser Art der Halterung kann es relativ leicht zum unbeabsichtigten Lösen des Ventilgehäuses kommen.

Allen vorstehenden Vorrichtungen ist gemein, dass sie keinen sicheren Sitz des Sprechventils und/oder Wärme-Feuchtigkeitstauschers gewährleisten, da bspw. beim Husten oder Niesen des Patienten erhebliche Drucke auftreten, die leicht Orkanstärke erreichen können, wodurch die Ventile regelrecht fortgeschleudert werden. Bis auf letztgenanntes Sprechventil weisen die bekannten Bauformen zusätzlich den Nachteil auf, dass sie sich beim Wechsel, bspw. zu Reinigungszwecken oder Austausch des Wärme-Feuchtigkeitstauschers, für den Patienten relativ schwer entnehmen lassen, weil der Hauptteil des Gehäuses von der Halterung umfangsmäßig erfasst ist.

Letzt genanntes Problem wird durch eine in EP 1 787 671 B1 beschriebene Vorrichtung gelöst, bei der ein im wesentlichen zylindrisches Gehäuse, das das Sprechventil und/oder den Wärme-Feuchtigkeitstauscher trägt, respektive aufnimmt, vorgesehen ist, wobei das Gehäuse am oder im Bereich seines proximalen Endes inwändig mit wenigstens zwei Nasen oder wenigstens einem Steg versehen ist, in die Haken, die im Bodenbereich eines Halterings vorgesehen sind, durch Verdrehung eingreifen, wobei zwischen den proximalen Anlageflächen des Gehäuses und kongruent zu diesen vorgesehenen Auflageflächen des Halterings im gegeneinander verspannten Zustand von Gehäuse und Haltering Dichtflächen gebildet sind. Bei dieser Lösung sind das Gehäuse und der Haltering, sowie die Haken aus einem steifen und wenig elastischen Material gefertigt und der Haltering mit einem Flansch, der mit dem Tracheostomapflaster verschweißt ist, versehen ist, wobei der Flansch seinerseits so dünn ausgeführt ist, dass er wieder elastisch ist. Diese Lösung garantiert zwar einen sicheren Sitz des Sprechventils und/oder des Wärme-Feuchtigkeitstauschers auf dem Tracheostomapflaster und angenehme Trageeigenschaften beim Patienten, erfordert aber weiterhin einen relativ hohen Fertigungsaufwand und einer gewissen Übung des Patienten beim Wechsel des Tauschers.

In EP 0 959 927 B1 ist eine Atemschutzausrüstung beschrieben, deren wesentlichstes Ziel es ist, einen Wärme-Feuchtigkeitstauscher auf einem Tracheostomapflaster zu schaffen, der den seitlichen Luftaustritt ermöglicht und dadurch Verunreinigungen durch anliegende Bekleidungsteile auf seiner Frontfläche vermeidet. Dazu wird besagte Frontfläche in einer Ausführungsform körperabseitig mit einer geschlossenen steifen Platte überdeckt und mit der anderen Seite direkt auf das Tracheostomapflaster aufgeklebt. Da die Oberflächen von Tracheostomapflastern relativ stark profiliert sind, eben wie bei üblichen Pflastern, lässt sich durch die in EP 0 959 927 B1 beschriebene Lösung des Aufklebens nur eine punktuelle Befestigung des Wärme-Feuchtigkeitstauschers realisieren, die einen sichern festen Halt nicht garantiert. Zusätzlich besteht bei dieser Lösung die Gefahr, dass beim Aufdrücken eines neuen Wärme-Feuchtigkeitstauschers (beim öfter erforderlichen Wechsel) auf das am Patienten befindlichen Tracheostomapflasters der Wärme-Feuchtigkeitstauscher in das Stoma des Patienten hinein gedrückt wird. Soll eine derartige Ausführung auch als Sprechventil Verwendung finden, wird in dieser Schrift vorgeschlagen, den Wärme-Feuchtigkeitstauscher zylindrisch so auszuführen, dass er von einem kurzen Zylinderstück, das in nicht näher beschriebener Weise mit dem Tracheostomapflaster verbunden ist, mittig aufgenommen wird. Damit wäre eine Funktion als Sprechventil gegeben, wenn die dortige steife Deckplatte auf den Zylinderkonus gedrückt wird. Auch dabei wird der Zylinderkonus in das Stoma des Patienten gedrückt, was von diesem als höchst unangenehm empfunden werden dürfte.

Außerdem sind aus einem etwas entfernter liegenden Stand der Technik, wie bspw. in DE 100 07 623 C1, WO 94/01158 A1 und DE 20 2005 008 359 U1 beschrieben, Lösungen zur Befestigung von Baugruppen, wie Sprechventilen und/oder Wärme-Feuchtigkeitstauschern, bekannt, wobei sich diese jedoch ausschließlich auf deren Befestigung an Trachealkanülen beziehen. Da bei solchen Verbindungen aber relativ starre Partner (Trachealkanüle und die dort anzubringende Baugruppe) miteinander verbunden werden, spielen dort Dichtigkeitsprobleme, im Gegensatz zur Anbringung genannter Baugruppen auf Tracheostomapflastern, praktisch keine Rolle.

Vorliegender Erfindung liegt daher die Aufgabe zugrunde, eine Halterung für Sprechventile und/oder Wärme-Feuchtigkeitstauscher für Laryngetomierte oder Tracheotomierte anzugeben, die zum einen einen festen Sitz eines Wärme-Feuchtigkeitstauschers und eine flache Bauform und zum anderen eine problemlose Austauschbarkeit durch den Patienten gewährleistet, wobei ein Eindrücken des Wärme-Feuchtigkeitstauschers beim Wechsel und bei der Benutzung als Sprechventil in das Tracheostoma des Patienten vermieden werden soll, wenn genannte Baugruppen auf einem Tracheostomapflaster befestigt werden. Darüber hinaus soll die erfindungsgemäße Lösung eine preiswerte Fertigung mit einem Minimum an einzelnen Komponenten gewährleisten und zugleich einen hohen Tragekomfort für den Patienten aufweisen.

Die Aufgabe wird durch die Merkmale des Patentanspruchs 1 und 7 gelöst. Vorteilhafte weitere Ausgestaltungen sind Gegenstand der nachgeordneten Ansprüchen.

Die erfindungsgemäße Halterung zeichnet sich dadurch aus, dass die Halterung für Sprechventile und/oder Wärme-Feuchtigkeitstauscher, eine elegante und ausgesprochen flache Bauform mit hohem Tragekomfort aufweist und dem Patienten einen einfachen Wechsel des Wärme-Feuchtigkeitstauschers ohne aufwändigere Manipulationen außerhalb seines Sichtbereichs ermöglicht. Zugleich gewährleistet die erfindungsgemäße Art der Befestigung des Wärme-Feuchtigkeitstauschers dessen festen Sitz auch bei höheren Druckbelastungen (wie bspw. beim Husten oder Niesen) und durch die elastische distale Oberflächenausbildung eine schmiegsame Anpassung an darüber getragene Kleidungsstücke und so eine gegenüber dem Stand der Technik erheblich ästhetischere Erscheinung.

Gegenüber bekannten Halterungen für den gleichen Zweck weist die erfindungsgemäße Lösung eine ausgesprochen komfortable Handhabung für den Patienten auf. Dieser kann das vollständige Sprechventil und/oder den Wärme-Feuchtigkeitstauscher durch einfaches Abziehen vom Träger entfernen, was für Reinigungszwecke oder Filterwechsel mehrmals täglich geschehen muss, wohingegen im befestigten Zustand eine absolut sichere festsitzende Arretierung gewährleistet ist. Dadurch, dass der wechselbare Filterkörper selbst, bis auf die hier erforderlichen Kaschierungen und Klebefläche, in seiner Grundausführung keine weiteren Bauteile erfordert, kann er ausgesprochen preiswert hergestellt werden, so dass sich bei einer solchen Ausführung nicht einmal seine Reinigung lohnt. Im Rahmen der Erfindung ebenfalls einsetzbare, aufwändiger aufgebaute Wärme-Feuchtigkeitstauscher lassen sich durch die neue Art ihrer Befestigung am Tracheostomapflaster in gleicher Weise problemlos wechseln und können ggf. einer Reinigung und Wiederverwendung zugeführt werden.

Nachfolgend einige Ausführungsbeispiele und schematische Zeichnungen.

Es zeigen:
- Fig. 1a: eine grundsätzliche Ausführung des bei vorliegender Erfindung eingesetzten Tracheostomapflasters mit einem Wärme-Feuchtigkeitstauscher in Explosionsdarstellung;
- Fig. 1b: eine grundsätzliche Ausführung des bei vorliegender Erfindung eingesetzten Tracheostomapflasters nach Fig. 1 a mit aufgesetztem Wärme-Feuchtigkeitstauscher im verbauten Zustand;
- Fig. 2a: eine bevorzugte Ausführungsform des mit einem Wärme-Feuchtigkeitstauscher versehbaren Tracheostomapflasters in Explosionsdarstellung;
- Fig. 2b: eine bevorzugte Ausführung des bei vorliegender Erfindung eingesetzten Tracheostomapflasters nach Fig. 2a mit aufgesetztem Wärme-Feuchtigkeitstauscher im verbauten Zustand;
- Fig. 3: eine grundsätzliche Ausführung des bei vorliegender Erfindung eingesetzten Wärme-Feuchtigkeitstauschers;
- Fig. 4a bis e: Beispiele möglich einsetzbarer Wärme-Feuchtigkeitstauscher und
- Fig. 5: eine weitere Ausbildungsmöglichkeit der bei vorliegender Erfindung zum Einsatz gelangenden Scheibe.

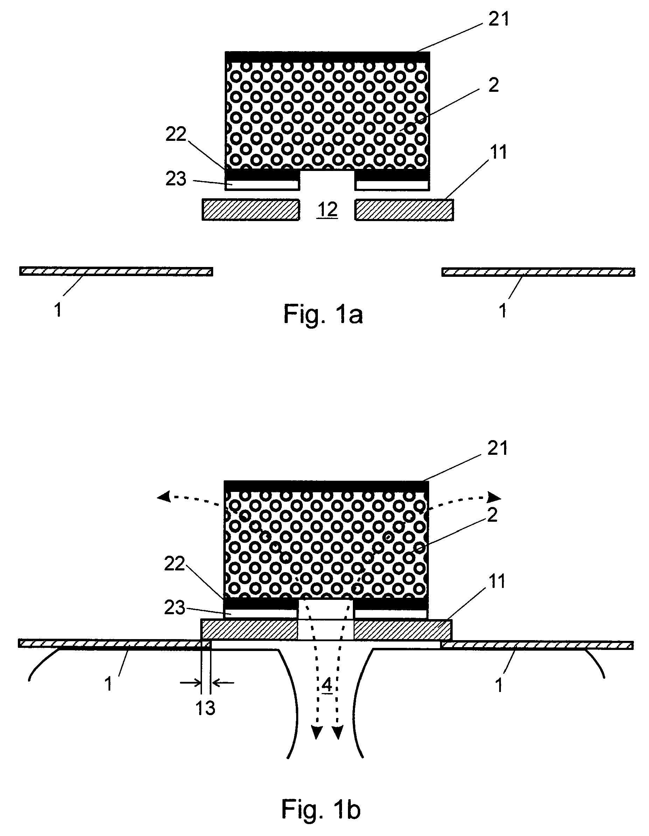

In Figur 1 a ist schematisch eine erste grundsätzliche Ausbildungsmöglichkeit des bei vorliegender Erfindung eingesetzten Tracheostomapflasters mit einem Wärme-Feuchtigkeitstauscher in Explosionsdarstellung in einer geschnittenen Ansicht dargestellt. Ein an sich übliches Tracheostomapflaster 1 wird im Rahmen der Erfindung mit einer Scheibe 11, bevorzugt einer Ringscheibe, bevorzugt durch Ultraschallschweißen, unlösbar im äußeren Randbereich 13 verbunden, wie es aus Figur 1 b ersichtlich ist. Die Ringscheibe weist eine Durchlassöffnung 12 mit einem Durchmesser von bspw. 9 mm auf. Für die Ringscheibe 11 kommen dabei in der Medizintechnik gängige Materialien wie PE (Polyethylen), TPE (thermoplastisches Elastomer) oder Vergleichbare, die einem Schweißvorgang zugänglich sind, in Betracht. Durch die Materialwahl und Dicke der Ringscheibe wird erreicht, dass die Ringscheibe 11 einerseits weich genug ist, um einem bevorzugten Ultraschallschweißen zugänglich zu sein und andererseits hart genug, um als biegesteife plane Klebeplatte für den anschließend aufzubringenden Wärme-Feuchtigkeitstauscher 2 zu sein. Bei ersten Versuchen zeigte sich, dass Dicken der Ringscheibe 11 von 1 mm bis 2,5 mm dem angestrebten Ziel grundsätzlich genügen.
Figur 1 b zeigt am ersten Beispiel beispielhaft den Randbereich 13 mit dem die Ringscheibe 11 mit dem Tracheostomapflaster 1 verbunden und über dem Stoma 4 platziert angeordnet ist, wobei die Ausnehmungen im Tracheostomapflaster 1 und der Ringscheibe 11 Durchlassbereiche bilden und den Luftaustausch zum Wärme-Feuchtigkeitstauscher 2 gewährleisten. Auf die durch die Ringscheibe 11 gebildeten planen Fläche wird anschließend ein Wärme-Feuchtigkeitstauscher 2 aufgesetzt, der an seiner der Ringscheibe 11 zugewandten Seite zumindest mit einer ringförmigen Klebefläche 23 versehen ist. Diese Klebefläche 23 ist so ausgeführt, dass sie ausschließlich den Kontaktbereich der Ringscheibe 11 erfasst, die frei liegende Luftdurchtrittsöffnung 12 also nicht behindert. Im Rahmen vorliegender Erfindung sollen dabei nur Wärme-Feuchtigkeitstauscher 2 zum Einsatz gelangen, die an ihrer stomaabseitigen Oberfläche durchgehend mit einer luftundurchlässigen Oberflächenkaschierung 21 versehen sind, wodurch die Luftaustauschwege (vgl. strichlinierte Pfeile in Fig. 1 b) seitlich durch den Wärme-Feuchtigkeitstauscher 2 gewährleistet sind, nicht jedoch durch besagte stomaabseitige Oberfläche.

In weiterer Ausgestaltung der Erfindung ist es vorteilhaft, genannte Klebefläche 23 nicht direkt auf den Wärme-Feuchtigkeitstauscher 2 aufzubringen, sondern vorher auch diese Seite des Wärme-Feuchtigkeitstauschers 2 mit einer kreisringförmigen luftundurchlässigen Oberflächenkaschierung 22 zu versehen. Diese kann analog zur Oberflächenkaschierung 21 ausgeführt und durch eine Dichtfolie, Oberflächenverhautung (bspw. durch thermische Behandlung des Filtermaterials) etc. gebildet sein. Wesentlich soll nur ihre Funktion der Luftdichtheit bei gleichzeitig erhaltener Elastizität und Geschmeidigkeit bei Druckbeaufschlagung sein. Wenn im Weiteren von Oberflächenkaschierung gesprochen wird, sollen alle Ausführungen, die vorstehender Maßgabe folgen, darunter verstanden sein und unter vorliegende Erfindung fallen.

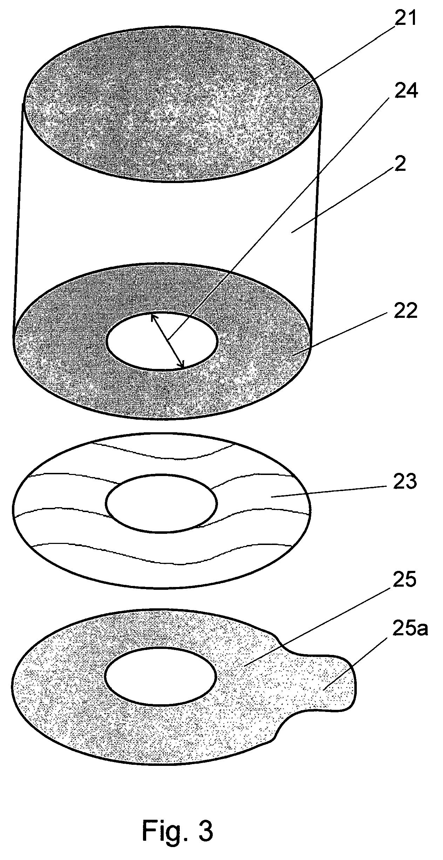

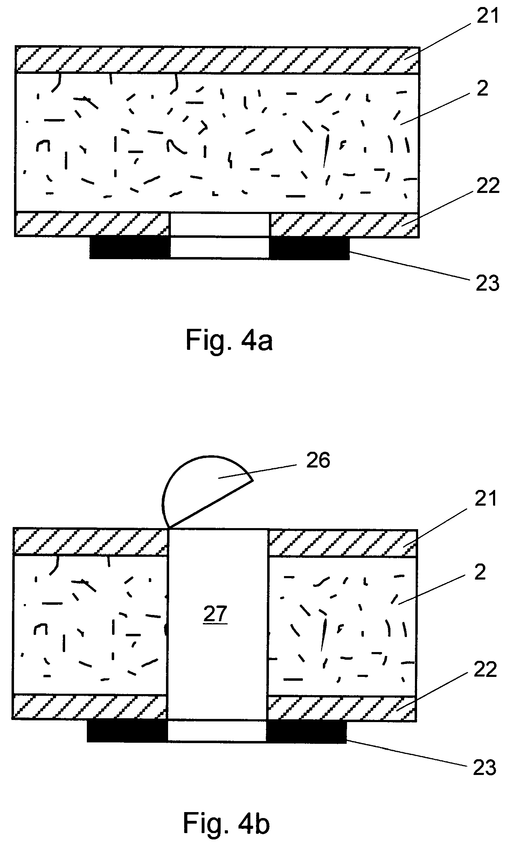

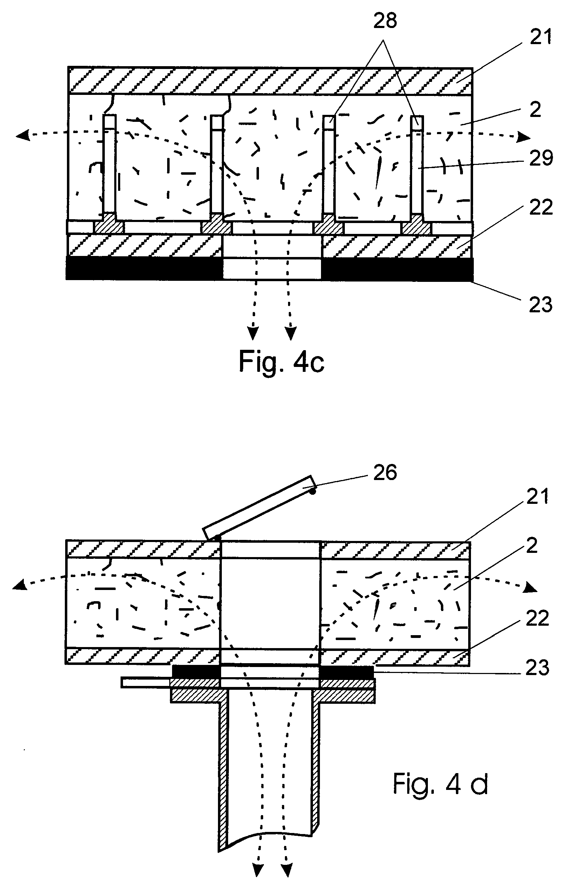

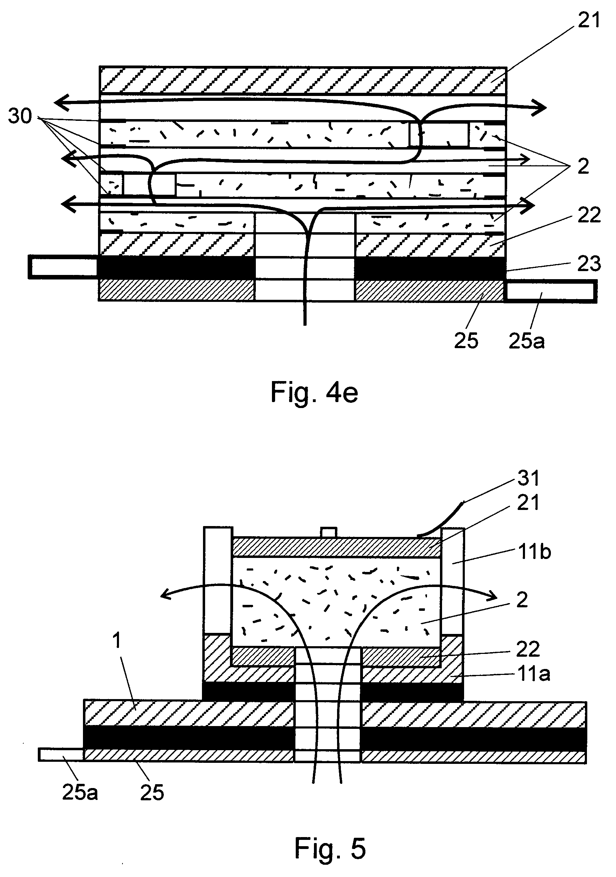

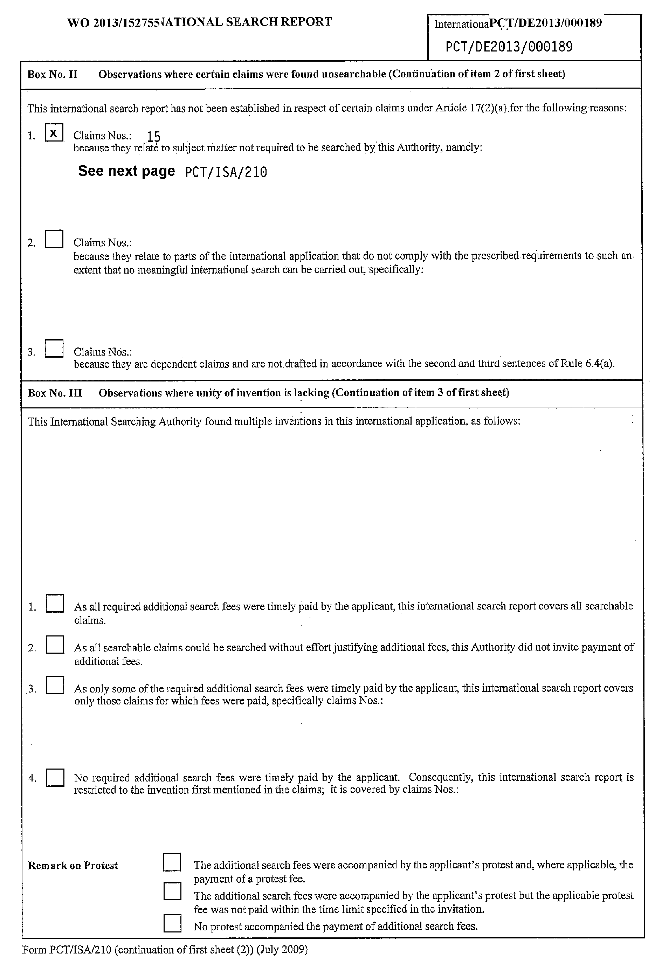

Nähere Angaben zur bevorzugten Ausgestaltung eines solchen im Rahmen der Erfindung zum Einsatz gelangenden Wärme-Feuchtigkeitstauschers erfolgen weiter unten anhand der speziellen Beschreibung zu den Figuren 3 und 4.

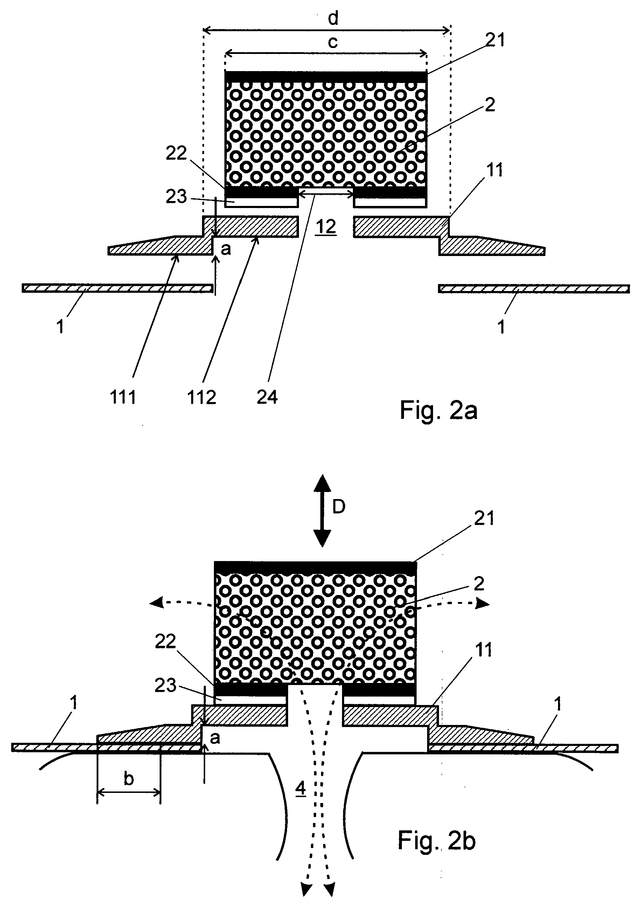

Zunächst wird anhand der Figuren 2a und 2b eine bevorzugte Ausführung des bei vorliegender Erfindung eingesetzten Tracheostomapflasters näher beschrieben, die der technologischen Herstellung leicht zugänglich ist. Dabei zeigt Fig. 2a eine bevorzugte Ausführungsform des mit einem Wärme-Feuchtigkeitstauscher versehbaren Tracheostomapflasters in Explosionsdarstellung und Fig. 2b die gleiche Ausführung im verbauten Zustand. In diesem Beispiel ist die Ringscheibe 11 gekröpft derart ausgebildet ist, dass sie einen außerhalb des Stomas 4 vorgesehenen äußeren Umfangsbereich b aufweist, der derart abgedünnt ausgeführt ist, dass er mit dem Tracheostomapflaster 1, bevorzugt durch Ultraschallschweißen, unlösbar verschweißbar ist. Dem abgedünnten Umfangsbereich b sind dabei im Beispiel Dicken zwischen 0,3 mm bis 0,7 mm gegeben, wobei er selber im Beispiel über eine Breite von ca. 4,6 mm mit dem Tracheostomapflaster unlösbar rundherum verschweißt ist. Wohingegen der Teil der Ringscheibe 11, der ausschließlich der Aufnahme des Wärme-Feuchtigkeitstauschers 2 dient, dicker ausgeführt ist, als der mit dem Tracheostomapflaster unmittelbar verbundene Ringscheibenbereich b, und zwar so dick, dass er eine hinreichende Biegesteifigkeit bei Druckbelastung auf den Wärme-Feuchtigkeitstauscher 2 beim Austausch oder Auslösung der Sprechfunktion aufweist. Im Beispiel haben sich dabei Dicken für genannten dickeren Ringscheibenbereich von ca. 1,5 mm als optimal erwiesen. Konkrete Dicken hängen jedoch vom für die Ringscheibe eingesetzten Material ab, wie Vorstehend bereits erwähnt.
Ohne, dass dies in den beispielhaften Figuren näher ersichtlich ist, sollen alle Kanten und Ecken der eingesetzten Ringscheiben 11 verrundet aufgeführt sein.
Im Rahmen dieses Beispiels hat sich als vorteilhaft erwiesen, dass bei gekröpfter Ausführung der Ringscheibe 11 diese bevorzugt so ausgeführt ist, dass zwischen der unteren Verschweißungsebene 111 der Ringscheibe 11 mit dem Tracheostomapflaster 2 und der unteren Ebene 112 des dicker ausgeführten Ringscheibenbereichs ein lichter Abstand a gebildet ist, der bevorzugt in der Größenordnung von
0,7 mm festgelegt ist. Damit ist gewährleistet, dass bei Druckbeaufschlagung D auf den Wärme-Feuchtigkeitstauscher 2 (vgl. Doppelpfeil in Fig. 2b) bei Wechsel des Wärme-Feuchtigkeitstauschers oder zum Erzielen der Sprechfunktion keine Druckbelastung des Stomas selbst oder ein Hineindrücken des Wärme-Feuchtigkeitstauschers 2 in das Stoma 4 auftreten kann.

Im Rahmen der Erfindung ist es weiterhin besonders vorteilhaft, den Außendurchmesser c des Wärme-Feuchtigkeitstauschers 2 etwas kleiner als Außendurchmesser d der Ringberandung festzulegen, was ein taktiles Ertasten und Zentrieren des aufzusetzenden Wärme-Feuchtigkeitstauschers 2 durch den Patienten auch ohne Sichtkontakt erleichtert, wie es in Fig. 2a angegeben ist. Gleiches gilt, wenn andere Geometrien als kreisförmige zum Einsatz gelangen sollen.

Die im Rahmen der Erfindung zum Einsatz gelangenden Wärme-Feuchtigkeitstauscher 2 können vielfältige konkrete Ausführungen aufweisen. Wesentlich ist, dass alle diese hier eingesetzten Ausführungen eine seitliche Luftzirkulation durch den Wärme-Feuchtigkeitstauschkörper ermöglichen und er an seiner distalen Oberfläche mit einer ganzflächigen, luftundurchlässigen elastischen Oberflächenkaschierung 21 und an seiner proximalen gegenüberliegenden Oberfläche zumindest mit einer ringförmigen Klebefläche 23 versehen ist. Bevorzugt soll der zum Einsatz gelangende Wärme-Feuchtigkeitstauschkörper 2 an der der aufnehmenden Ringscheibe 11 zugewandten Seite mit einer luftundurchlässigen Oberflächenkaschierung 22 mit kreisförmiger, einen Luftdurchlass ermöglichenden Ausnehmung 24 versehen sein, wobei die so gebildete Ringfläche der Oberflächenkaschierung mit einer Klebefläche 23 beschichtet ist, die vor dem Aufsetzen auf den biegesteifen Bereich der Ringscheibe 11 mit einer abziehbaren Schutzfolie 25 mit integrierten Anfasser 25a versehen ist, wie es schematisch Fig. 3 in Explosionsdarstellung zeigt. Wenn im Vorstehenden immer von zylindrischen, kreisförmigen oder kreisringförmigen Gestaltungen des Wärme-Feuchtigkeitstauschkörpers 2 gesprochen wurde, entspricht dies den bevorzugten Ausbildungen. Im Rahmen der Erfindung sind jedoch ausdrücklich auch andere Ausformungen, wie ovale und rechteckige mit entsprechenden korrespondierenden Gestaltungen der beanspruchten Ringscheiben 11 und deren Durchlässe 12, sowie damit korrespondierenden Klebe- 23 und Kaschierflächen 22 mit erfasst.

Sind vorstehende Maßgaben eingehalten, können die Wärme-Feuchtigkeitstauschkörper auch nach folgenden speziellen Beispielen und besonderen weiteren Ausbildungen des Wärme-Feuchtigkeitstauschkörpers 2 selbst gefertigt sein, wie im Nachstehenden beispielhaft dargestellt.

Dazu zeigt Fig. 4a beispielhaft einen Feucht-Wärme-Austauscher 2, der in Form einer runden, eckigen oder ovalen Schaumstoffform ausgebildet sein kann, welcher zur Oberflächenkaschierung 21, 22 mit einer elastischen Dichtfolie 21 an der äußeren Oberfläche und mit einer mit einem Durchgangsöffnung versehenen Dichtfolie 22 an der gegenüberliegenden Oberfläche versehen ist. Ein Klebering 23 ermöglicht die Konnektion mit einem Tracheostomapflaster oder einem Trachealkanülenschild (in Fig. 4a nicht dargestellt). Mittels Fingerdruck kann der Schaumstoff mit der oberen Folie auf die Öffnung der Innenkanüle oder des Stomas gepresst werden, was den Atem im Feucht-Wärme-Austauscher umleitet und eine Sprechfunktionen ermöglicht. Die Verbindung zwischen einem Tracheostomapflaster oder einem Kanülenflansch und dem Feucht-Wärme-Austauscher kann auch durch eine Klettverbindung erreicht werden, die den Feucht-Wärme-Austauscher jederzeit lösbar und durch einen neuen unkompliziert ersetzen lässt. Dadurch wird in beiden Anwendungsfällen die Bauhöhe extrem verringert und die Atemverluste zwischen einem eventuell noch vorgesehenem Gehäuse (vgl. weiter unten) zur Filteraufnahme und dem Filter auf Null reduziert. Nach dem Lösen des nicht dargestellten Fingers von der Oberfläche der Dichtfolie des Feucht-Wärme-Austauschers ist der Atemweg durch die entspannende Federwirkung des Schaumstofffilters wieder frei.

Als Beispiel des Feucht-Wärme-Austauschers ist es weiterhin möglich, wie in Fig. 4b beispielhaft skizziert, zur Regulierung des Entspannungsdruckes des Schaumstofffilters 2, zur Optimierung des Atemwiderstandes und der Luftbefeuchtung einen Filterkern 27 vorzusehen, der aus einem anderen Filterstoff als dem des Schaumstofffilters 2 besteht. Dieser Filterkörper kann im Durchmesser auch geringer ausgeführt sein, als die zur Atmung vorgesehene Durchgangsöffnung Richtung Stoma. Dabei wird durch den Filterkörper eine Atemhülse gebildet, die eine Schleimbildung ermöglicht und im Zentrum durch eine Druckplatte 26 abgedeckt ist. Wird die Druckplatte 26 mittels Fingerdruck auf die gegenüberliegende Aufsitzfläche gedrückt, kann der Patient einen Sprechvorgang einleiten, da der Atemweg durch den Schaumstofffilter 3 versperrt ist. Zusätzlich ermöglicht in dieser Variante eine kleiner ausgeführte Druckplatte 26 (in Fig. 4b im eröffneten Zustand dargestellt) eine separate Entnahme des inneren Filterkörpers und damit eine gesonderte Schleimbeseitigung.
Eine weitere Variante nach Fig. 4c skizziert die Möglichkeit, den Schaumstoffkörper 2 mit einer inwändigen Stabilisierung 29 in Schlitzen 28 zu versehen. Eine solche Stabilisierung 29 dient dazu, dass bei Druck auf den Schaumstoffkörper 2 keine Flüssigkeit aus dem Filter seitlich herausgepresst wird.

Figur 4d zeigt ein Beispiel eines einsetzbaren Wärme-Feuchtigkeitstauschers 2, der wiederum aus einem Filterschaumstoff besteht, der auf beiden Oberflächen mit einer transparenten Folie, sprich mit einer Kaschierung 21, 22, versehen ist. In diesem Beispiel ist im Zentrum ein zylindrischer Atemraum geschaffen, der mittels einer Druckplatte 26, die hier als Sicherungsklappe ausgeführt ist, verschlossen gehalten werden kann. Bei einem Hustenstoß öffnet sich diese Klappe und der Druck kann entweichen, ohne den festen Sitz des Wärme-Feuchtigkeitstauschers 2 zu gefährden. Anschließend kann die Klappe wieder verschlossen werden und ermöglicht das Atmen entsprechend der in Fig. 4d durch strichlinierte Doppelpfeile dargestellten Wege. Des Weiteren können beim Öffnen der Klappe der geschaffene Atemraum und als auch das Innere der in diesem Beispiel gezeigten Trachealkanüle gesäubert werden.

Auch in diesem speziellen Beispiel ist der Filterschaumstoff mit seiner transparenten Folienkaschierung mit einer Klebescheibe 23 ausgerüstet, die durch Abziehen der Schutzfolie mittels Anfasser auf der Trachealkanüle mit entsprechend gestaltetem Innenkanülenflansch lösbar befestigt werden kann. Somit kann auch hier der Filterschaumstoff jederzeit erneuert werden. Auch bei einer derartigen Verwendung des Wärme-Feuchtigkeitstauschers wird eine geringe Bauhöhe erreicht und der Tragekomfort für den Patienten erhöht. Der Filterschaumstoff kann, wie in allen anderen Ausführungsformen ebenfalls, verschiedene Atemwiderstände durch unterschiedliche Porendichte aufweisen.

Ebenso weist ein Wärme-Feuchtigkeitstauscher die damit beabsichtigten Vorteile auf, wenn ihm in sich selber einen völlig anderen Aufbau, wie in Fig. 4e dargestellt, gegeben ist. In diesem Beispiel sind im Filterschaumstoff 2 perforierte, antimikrobielle Schichten oder Labyrinthschichten in antimikrobielle Folien oder Textilgeweben, welche mit Zink oder Nanosilber ausgerüstet sind, eingebettet, die durch Gestänge, Gehäuse oder Klebestreifen 30 fixiert sind. Die Perforationen bzw. das Labyrinth sind so ausgebildet, dass der Atemstrom ausreichend und nicht behindernd für den Patienten wirkt, indem er über die antimikrobiellen Schichten streicht und damit die Bakterien abgetötet werden. Der Atemweg wird auch hier seitlich durch den Schaumstoff und die Labyrinthöffnungen geleitet. Gemäß der Erfindung ist auch hier eine Klebeschicht 23 vorgesehen, die mittels einer Schutzfolie 25 vor Einbau geschützt ist, welche sich mit einem Anfasser 25a abziehen und der gesamte Wärme-Feuchtigkeitstauscher somit auf ein Tracheostomapflaster oder eine Kanüle mit entsprechender Aufnahmeplatte geklebt werden kann.

Schließlich zeigt Fig. 5 eine weitere Variante, die eine Modifizierung der erfindungsgemäß vorgesehenen Scheibe auf dem Tracheostomapflaster 1 andeutet. In diesem Beispiel ist das Pflaster 1 mit einer profilierten Platte 11a verbunden, die eine gehäuseartige Ausformung 11 b aufweist, die seitlich in Fig. 5 nicht näher erkenntliche Luftdurchtrittsöffnungen aufweist. Ein wie auch immer gestalteter Wärme-Feuchtigkeitstauscher 2, der mit bislang schon näher beschriebenen oberen und unteren flexiblen Oberflächenverhautungen 21, 22 versehen ist, kann in dieses Gehäuse eingelegt und dort befestigt und mittels eines Anfassers 31 leicht entnommen werden. In gewisser Weise geht der Vorteil der geschmeidigen Anpassung der an darüber getragene Bekleidungsstücken bei dieser Ausführung, je nach Ausführung der Ausformung 11 b, verloren, jedoch bleiben die übrigen Vorteile erhalten. Dieses Beispiel soll nur dazu dienen, um zu zeigen, dass die erfindungsgemäß auf dem Tracheostomapflaster 1 vorgesehene Scheibe weiteren Gestaltungsmöglichkeiten im Rahmen der Erfindung unterliegen kann.

Es liegt selbstverständlich im Rahmen der Erfindung, vorgeschlagene Wärme-Feuchtigkeitstauschkörper mit beidseitig angebrachten luftundurchlässigen, flexiblen elastischen Oberflächenkaschierungen auch auf Trachealkanülen, Tuben etc. mit entsprechenden planen Aufnahmeflächen zu verwenden, so dass auch für diese Verwendungen keine gesonderten Wärme-Feuchtigkeitstauscher vorgehalten werden müssen.

### Bezugszeichenliste

- 1: - Tracheostomapflaster
- 11: - Ringscheibe
- 11a: - profilierte Platte
- 11 b: - gehäuseartige Ausnehmung
- 111: - untere Verschweißungsebene
- 112: - untere Ebene des dicker ausgeführten Ringscheibenbereichs
- 12: - Durchlassöffnung der Ringscheibe
- 13: - verbundener Randbereich
- 2: - Wärme-Feuchtigkeitstauscher
- 21: - luftundurchlässige Oberflächenkaschierung
- 22: - kreisringförmige, luftundurchlässige Oberflächenkaschierung
- 23: - ringförmige Klebefläche
- 24: - Ausnehmung
- 25: - Schutzfolie
- 25a: - Anfasser
- 26: - Druckplatte
- 27: - Filterkern
- 28: - Schlitze
- 29: - Stabilisierung
- 30: - Klebestreifen
- 31: - Entfernungslasche
- a: - lichter Abstand
- b: - verbundener Ringscheibenbereich
- c: - Außendurchmesser des Wärme- Feuchtigkeitstauschers
- d: - erfassbarer Außendurchmesser der Ringscheibe
- D: - Doppelpfeil - Druckbeaufschlagung

## Patentansprüche

1. Halterung für Sprechventile und Wärme-Feuchtigkeitstauscher und dafür geeignete Wärme-Feuchtigkeitstauscher, bei denen ein mit seitlicher Luftzufuhr versehener Wärme-Feuchtigkeitstauscher (2) über einem Tracheostomapflaster (1) lösbar aufgebracht ist, **dadurch gekennzeichnet, dass** ein Tracheostomapflaster (1) im Randbereich (13, b) seiner Durchlassöffnung fest und unlösbar mit einer das Stoma (4) überdeckenden und mit einer Luftdurchtrittsöffnung (12) versehenen Scheibe (11) hinreichender Biegesteifigkeit bei Druckbelastung auf den Wärme-Feuchtigkeitstauscher (2) beim Austausch oder Auslösung der Sprechfunktion, verbunden ist, auf die ein Wärme-Feuchtigkeitstauscher (2) lösbar aufklebbar ist, der an seiner stomaabseitigen Oberfläche durchgehend mit einer luftundurchlässigen Oberflächenkaschierung (21) und an seiner der Scheibe (11) zugewandten Seite zumindest mit einer umlaufenden Klebefläche (23) versehen ist.

2. Halterung nach Anspruch 1, **dadurch gekennzeichnet, dass** die Scheibe (11) als Ringscheibe ausgeführt ist.

3. Halterung nach Anspruch 1 oder 2, **dadurch gekennzeichnet, dass** die Scheibe (11) gekröpft derart ausgebildet ist, dass sie einen außerhalb des Stomas (4) vorgesehenen äußeren Umfangsbereich (b) aufweist, der derart abgedünnt ausgeführt ist, dass er mit dem Tracheostomapflaster (1), bevorzugt durch Ultraschallschweißen, unlösbar verschweißt ist.

4. Halterung nach Anspruch 3, **dadurch gekennzeichnet, dass** der Teil der Ringscheibe (11), der ausschließlich der Aufnahme des Wärme-Feuchtigkeitstauschers (2) dient, dicker ausgeführt ist, als der mit dem Tracheostomapflaster unmittelbar verbundene Ringscheibenbereich, und zwar so dick, in der Größenordnung von 1 mm bis 2,5 mm, dass er eine hinreichende Biegesteifigkeit bei Druckbelastung auf den Wärme-Feuchtigkeitstauscher (2) beim Austausch oder Auslösung der Sprechfunktion aufweist.

5. Halterung nach Anspruch 3 und 4, **dadurch gekennzeichnet, dass** die gekröpfte Ausführung der Ringscheibe (11) so ausgeführt ist, dass zwischen der unteren Verschweißungsebene (111) der Ringscheibe (11) mit dem Tracheostomapflaster (1) und der unteren Ebene (112) des dicker ausgeführten Ringscheibenbereichs ein lichter Abstand a gebildet ist, der bevorzugt in der Größenordnung von 0,7 mm festgelegt ist.

6. Halterung nach Anspruch 1, **dadurch gekennzeichnet, dass** die umlaufende Klebefläche (23) auf einer luftundurchlässigen Oberflächenkaschierung (22) mit bevorzugt kreisförmiger Luftdurchlassfläche oberhalb des Stomas (4) auf dem Wärme-Feuchtigkeitstauscher (2) vorgesehen ist.

7. Wärme-Feuchtigkeitstauscher für eine Halterung nach Anspruch 1, **dadurch gekennzeichnet, dass** ein beliebig ausgebildeter, eine seitliche Luftzirkulation ermöglichender Wärme-Feuchtigkeitstauschkörper (2) an seiner distalen Oberfläche mit einer ganzflächigen, luftundurchlässigen elastischen Oberflächenkaschierung (21) und an seiner proximalen gegenüberliegenden Oberfläche mit einer luftundurchlässigen Oberflächenkaschierung (22) mit einer, einen Luftdurchlass ermöglichenden Ausnehmung (24) versehen ist, wobei die so gebildete umlaufende Fläche der Oberflächenkaschierung mit einer Klebefläche (23) beschichtet ist, die vor dem Aufsetzen auf den biegesteifen Bereich der Scheibe (11) mit einer abziehbaren Schutzfolie (25) versehen ist.

8. Wärme-Feuchtigkeitstauscher nach Anspruch 7, **dadurch gekennzeichnet, dass** der Wärme-Feuchtigkeitstauscher (2) selbst durch einen öffenporigen, porösen zusammenpressbaren Schaumstoff gebildet ist.

9. Wärme-Feuchtigkeitstauscher nach Anspruch 7, **dadurch gekennzeichnet, dass** der Wärme-Feuchtigkeitstauscher (2) inwändig mit einem zusätzlichen, separat entnehmbaren Filterkern (27) versehen ist.

10. Wärme-Feuchtigkeitstauscher nach Anspruch 9, **dadurch gekennzeichnet, dass** über dem zusätzlichen Filterkern (27) eine verschließbare Druckplatte (26) vorgesehen ist.

11. Wärme-Feuchtigkeitstauscher nach Anspruch 7, **dadurch gekennzeichnet, dass** der Wärme-Feuchtigkeitstauscher (2) mit inwändigen Stabilisierungen (29) in Schlitzen (28) versehen ist.

12. Wärme-Feuchtigkeitstauscher nach Anspruch 7, **dadurch gekennzeichnet, dass** der Wärme-Feuchtigkeitstauscher (2) einen filterkörperfreien, mittigen Atemraum beinhaltet, der mittels einer Druckplatte (26), die als eröffenbare Sicherungsklappe ausgeführt ist, verschlossen gehalten ist.

13. Wärme-Feuchtigkeitstauscher nach Anspruch 7, **dadurch gekennzeichnet, dass** der Filterkörper des Wärme-Feuchtigkeitstauschers (2) in Form labyrinthartig geführter, beabstandeter Schichten ausgebildet sind, die mit antimikrobiellen Folien oder Beschichtungen ausgerüstet sind.

14. Wärme-Feuchtigkeitstauscher nach einem der vorstehenden Ansprüche, **dadurch gekennzeichnet, dass** der Außendurchmesser (c) des Wärme-Feuchtigkeitstauschers (2) etwas kleiner als Außendurchmesser (d) der Berandung der Scheibe (11) festgelegt ist.

## Claims

1. Mounting for voice valves and heat-moisture exchanger and heat-moisture exchanger suited therefor, to which a heat-moisture exchanger (2) having a lateral air supply is detachably fastened by a tracheostoma adhesive plaster (1), **characterized in that** a tracheostoma plaster (1) in its rim portion (13, b) of its passage opening is firmly and non-detachably connected to a disk (11), said disk covering a stoma (4) and being provided with an air passage opening (12), said disk having a sufficient flexural rigidity when the heat-moisture exchanger (2) is pressure loaded due to an exchange or starting of the voice function, the heat-moisture exchanger (2) is adapted to be detachably glued on to said disk (11), said heat-moisture exchanger (2) being provided on its surface offside to the stoma with an airtight surface coating (21) and on its side opposite to the disk (11) at least with a circumferential adhesive face (23).

2. Mounting as claimed in claim 1, **characterized in that** the disk (11) is embodied as an annular disk.

3. Mounting as claimed in claim 1 or 2, **characterized in that** the disk (11) is designed offset in a manner, that it has an external circumferential range (b) outside of the stoma (4) which is embodied thinned out in a manner that it is non-detachably welded to the tracheostoma plaster (1), preferably by ultrasonic welding.

4. Mounting as claimed in claim 3, **characterized in that** that portion of the annular disk (11) which is exclusively adapted for receiving the heat-moisture exchanger (2) is embodied thicker than the annular range of the disk which is directly connected to the tracheostoma plaster and the thickness being of an order of size within 1 mm to 2.5 mm so that it shows a sufficient flexural rigidity when the heat-moisture exchanger (2) is subject to a pressure load in the course of an exchange or starting of the voice function.

5. Mounting as claimed in claim 3 and 4, **characterized in that** the offset design of the annular disk (11) is such executed that a clear distance a forms between the lower welding plane (111) of the annular disk (11) with the tracheostoma plaster (1) and the lower plane (112) of the annular range being executed thicker, the clear distance a being preferably in the order of size of 0.7 mm.

6. Mounting as claimed in claim 1, **characterized in that** the circumferential adhesive face (23) is provided with an airtight surface coating (22) having a preferably circular air passage face above the stoma (4) on the heat-moisture exchanger (2).

7. Heat-moisture exchanger for a mount as claimed in claim 1, **characterized in that** a heat-moisture exchanger body (2) being embodied at will and permitting a lateral air circulation is provided at its distal surface with a surface coating (21) on its entire surface which is elastically and impermeable to air and on its proximal opposite surface with a surface coating (22) being impermeable to air and being provided with a recess (24) adapted to permit an air passage, whereby the circumferential surface of the surface coating formed in this way is coated with an adhesive face (23) which is covered with a shippable protective foil (25) before being applied to the flexural rigid part of the disk (11).

8. Heat-moisture exchanger as claimed in claim 7, **characterized in that** the heat-moisture exchanger (2) itself is made of a porous compressible foamed plastic with open pores.

9. Heat-moisture exchanger as claimed in claim 7, **characterized in that** the heat-moisture exchanger (2) is provided internally with an additional filter core (27) which can be taken out separately.

10. Heat-moisture exchanger as claimed in claim 9, **characterized in that** above the additional filter core (27) a lockable pressure plate (26) is provided.

11. Heat-moisture exchanger as claimed in claim 7, **characterized in that** the heat-moisture exchanger (2) is internally provided with stabilizers (29) in slots (28).

12. Heat-moisture exchanger as claimed in claim 7, **characterized in that** the heat-moisture exchanger (2) includes a central respirator chamber which is free of filter body material, said respirator chamber is kept closed by a pressure plate (26) which is embodied as a safety plate adapted to be opened.

13. Heat-moisture exchanger as claimed in claim 7, **characterized in that** the filter body of the heat-moisture exchanger (2) is embodied as layers guided in the shape of labyrinth sheets spaced to one another, which are furnished with antimicrobial foils or coatings.

14. Heat-moisture exchanger as claimed in one of the preceding claims, **characterized in that** the outer diameter (c) of the heat-moisture exchanger (2) is selected somewhat smaller than the outer diameter (d) of the rim portion of the disk (11)

## Revendications

1. Le dispositif destiné à assurer la fixation de valves de phonation et d'échangeurs de chaleur et d'humidité appropriés, parmi lesquels un échangeur de chaleur et d'humidité (2) doté d'une possibilité d'insufflation latérale de l'air, étant disposé de manière détachable au-dessus d'un pansement de trachéostomie, est **caractérisé en ce qu'** un pansement de trachéostomie (1) dans la zone du pourtour (13, b) de son orifice de passage de l'air est relié de manière solidaire et indétachable à une rondelle (11) recouvrant la stomie (4) et pourvue d'une ouverture permettant la circulation de l'air (12) et présentant une rigidité suffisamment grande à la flexion en cas d'une pression excercée sur l'échangeur de chaleur et d'humidité (2) au moment du changement ou du déclenchement de la phonation, rondelle sur laquelle il sera possible de coller de manière détachable un échangeur de chaleur et d'humidité (2) qui sur sa surface opposée à la stomie est doté partout d'un doublage superficiel (21) étanche à l'air et sur le côté en face de la rondelle (11) au moins d'une surface adhésive circulaire (23).

2. Le dispositif de fixation selon la revendication 1 est **caractérisé en ce que** la rondelle (11) est une rondelle annulaire.

3. Le dispositif de fixation selon les revendications 1 ou 2 est **caractérisé en ce que** la rondelle (11) est coudée de sorte qu'elle présente une zone périphérique extérieure (b) située en dehors de la stomie (4) et étant amincie de sorte qu'il soit possible de la souder de manière indétachable, de préférence par soudage aux ultrasons, sur le pansement de trachéostomie (1).

4. Le dispositif de fixation selon la revendication 3 est **caractérisé en ce que** la partie de la rondelle (11) servant exlusivement de support pour l'échangeur de chaleur et d'humidité, est plus épaisse que la zone circonférentielle extérieure reliée directement au pansement de trachéostomie, de l'ordre de 1 mm à 2,5 mm, pour pouvoir assurer une rigidité suffisamment grande a la flexion en cas de charge de pression agissant sur l'échangeur de chaleur et d'humidité (2) au moment de son changement ou du déclenchement de la phonation.

5. Le dispositif de fixation selon les revendications 3 et 4 est **caractérisé en ce que** la rondelle (11) coudée est exécutée de sorte que l'écart entre le niveau de la zone de soudage inférieure (111) de la rondelle (11) portant le pansement de trachéostomie (1) et le niveau inférieur (112) de la zone plus épaisse de la rondelle devait s'élever à une distance a de l'ordre de 0,7 mm.

6. Le dispositif de fixation selon la revendication 1 est **caractérisé en ce que** la surface adhésive (23) circonférentielle est disposée sur un doublage superficiel (22) pourvue de préférence d'une surface circulaire de passage d'air, au-dessus de la stomie (4) sur l'échangeur de chaleur et d'humidité (2).

7. Echangeur de chaleur et d'humidité pour un dispositif de fixation selon la revendication 1, est **caractérisé en ce qu'**un échangeur de chaleur et d'humidité (2) de conception diverse, permettant une insufflation d'air latérale, est doté sur toute la surface distale d'un doublage superficiel élastique, étanche à l'air (21), et sur la surface proximale sur le côté opposé, d'un doublage superficiel (22) étanche à l'air ainsi que d'un évidement (24) permettant un passage d'air. La surface circulaire ainsi formée est recouverte d'une surface adhésive (23) dotée d'une feuille protectrice détachable (25) avant sa mise en place sur la zone rigide à la flexion de la rondelle (11).

8. Echangeur de chaleur et d'humidité selon la revendication 7 est **caractérisé en ce que** l'échangeur de chaleur et d'humidité (2) lui-même est fabriqué en mousse conçue de facon poreuse avec des pores ouvertes et pouvant être comprimée.

9. Echangeur de chaleur et d'humidité selon la revendication 7 est **caractérisé en ce que** l'échangeur de chaleur et d'humidité (2) est en plus muni dans son intérieur d'un noyeau filtrant (27) pouvant être retiré séparément.

10. Echangeur de chaleur et d'humidité selon la revendication 9 est **caractérisé en ce qu'**une plaque de pression pouvant être fermée (26) est placée au-dessus du noyeau filtrant intérieur (27) supplémentaire.

11. Echangeur de chaleur et d'humidité selon la revendication 7 est **caractérisé en ce que** l'échangeur de chaleur et d'humidité (2) est muni sur la paroi intérieure de stabilisations (29) placées dans des fentes (28).

12. Echangeur de chaleur et d'humdité selon la revendication 7 est **caractérisé en ce que** l'échangeur de chaleur et d'humdité (2) comprend dans son centre un espace de respiration exempte de corps filtrants pouvant être tenu fermé moyennant d' une plaque de pression (26) dotée d'un clapet de sécurité ouvrant.

13. Echangeur de chaleur et d'humdité selon la revendication 7 est **caractérisé en ce que** que le corps filtrant de l'échangeur de chaleur et d'humidité (2) étant conçu en forme de couches espacccées les unes des autres, disposées en forme de labyrinthe et dotées de feuilles ou revêtements antimicrobiens.

14. Echangeur de chaleur et d'humdité selon une des revendications précédentes est **caractérisé en ce que** le diamètre extérieur (c) de l'échangeur de chaleur et d'humidité (2) est conçu un peu plus petit que le diamètre extérieur de la bordure de la rondelle (11).
